# EUROPEAN PATENT SPECIFICATION

(11) **EP 1 331 939 B1**
(45) Date of publication and mention of the grant of the patent: **13.09.2006**
(21) Application number: 00991821.0
(22) Date of filing: 16.10.2000
(51) Int. Cl.: A61K 36/75, A61P 11/06

(54) **MURRAYA KOENIGII EXTRACTS FOR TREATING ASTHMA**
MURRAYA KOENIGII-EXTRAKTE ZUR BEHANDLUNG VON ASTHMA
EXTRAITS DE MURRAYA KOENIGII SERVANT A TRAITER L'ASTHME

(43) Date of publication of application: 06.08.2003
(73) Proprietor: Council of Scientific and Industrial Research;an Indian Reg. body incorporated under Reg. of Societies Act (Act XXI of 1860), New Delhi 110 001 (IN)
(72) Inventor: BHADRA, R., c/o Indian Institute of Chemical Biol., Calcutta 700 032 (IN); PAL, B., c/o Indian Institute of Chemical Biol., Calcutta 700 032 (IN); DAS, K., c/o Indian Institute of Chemical Biol., Calcutta 700 032 (IN); BHATTACHARAYA, S., c/o Indian Ins. of Chemical Bio, Calcutta 700 032 (IN)
(74) Representative: Fairbairn, Angus Chisholm
(86) International application number: PCT/IN2000/000102
(87) International publication number: WO 2002/032440

(56) References cited:
- SINGH Y N: "Traditional medicine in Fiji: some herbal folk cures used by Fiji indians" J ETHNOPHARMACOL, vol. 15, no. 1, 1986, pages 57-88, XP001000897
- PATENT ABSTRACTS OF JAPAN vol. 1997, no. 01, 31 January 1997 (1997-01-31) & JP 08 225420 A (INAHATA KORYO KK;NIPPON FUNMATSU YAKUHIN KK), 3 September 1996 (1996-09-03)
- PATENT ABSTRACTS OF JAPAN vol. 1995, no. 08, 29 September 1995 (1995-09-29) & JP 07 138126 A (MIKIMOTO PHARMACEUT CO LTD;OTHERS: 01), 30 May 1995 (1995-05-30)
- KHAN BEENA A ET AL: "Anti-oxidant effects of curry leaf, Murraya koenigii and mustard seeds, Brassica juncea in rats fed with high fat diet." INDIAN JOURNAL OF EXPERIMENTAL BIOLOGY, vol. 35, no. 2, 1997, pages 148-150, XP001000038 ISSN: 0019-5189 cited in the application

## Description

### Technical Field

The present invention relates to use of principles extracted from the plant *Murraya koenigii* for treatment of asthma.

The invention also provides novel compositions useful in the treatment of asthma

### Background Art

Respiratory- diseases such as asthma are reaching epidemic proportions in both the developed and developing world (Nature, Vol. 402, Supplement, and No. 6760, 1999. A special supplement on allergy and asthma). In countries such as Britain and Australia, the respiratory problem translates to 1 in 4 children under age of 14 years having asthma. The disease causes distress and misery in millions often at a time in their lives when they should be most active. Asthma interferes with sleep, intellectual functioning and recreational activities. At one extreme, asthma can be life threatening leading to occurrence of deaths that are avoidable.

Expert panel report has offered guidelines for diagnosis and management of asthma (JAMA Asthma Information Center, www.amaasssm.org/special/asthma/treatmnt/guide /guideline/ gudelin.htm). Control of factors contributing to asthma severity is another avenue for management (JAMA Asthma Information Center, www.amaasssm.org/special /asthma /treatmnt/guide/guidelin/comp2/comp2.htm). Several therapies and medications have also been suggested (JAMA Asthma Information Center, www.amaasssm.org/special/asthma/treatmnt/guide/guidelin/comp3/medicat/meditoc.htm and www.amaass.org/special asthma/treatmnt/guide/guidelin/comp3/comp3.htm). Immunotherapy is another_route for the treatment (JAMA Asthma Information Center, www.amasssm. org/special/asthma/treatmnt/guide/guidelin/comp2/immunoth.htm). Complementary alternative medicine is also being tried (JAMA Asthma Information Center,www.amaasssm.org/special/asthma/treatmnt/guide/guidelin/comp3/medicat/alternat .hmt). However, no satisfactory medication for cure of the disease has been found.

Herein the active factors in Murra*ya koenigii* was prepared and used under in vitro system to show its usefulness for relief, treatment and cure of asthmatic problem. The plant *Murraya koenigii* has been studied and reported to have various medicinal values (Chakraborty, M.P Phytochemistry 1997, Oct; Khan B. A, Indian J of Expt. Biol. 1997 Feb.; Khan B A Indian J of Physiology and Pharmacology 1996, Apr.; Khan BA Plant Foods Hum. Nutr. 1996 Jun).

The leaf of this plant is widely used in various food preparations in India. Further, the plant is ubiquitous. Thus the prior art suggests that the extracts of the plant have several medicinal and other properties which have been widely investigated. This is adequate proof that the extracts of the plant can be safely consumed by humans without any side effects.

The applicants undertook a study on the extracts of this plant to identify the therapeutic use of the principles of this plant. During their study, the applicants discovered the presence of certain active principles in the leaf of *Murraya koenigii* which surprisingly were found to be useful in the treatment and cure of asthma.

### Disclosure of the Invention

The main objective of the present invention is to provide a treatment of asthmatic conditions employing active principles extracted from the plant *Murraya koenigii* in combination with other herbal extracts.

Still another object is to provide novel compositions containing active principles extracted from the plant *Murraya koenigii* and useful in the treatment of asthma.

### Summary of the invention

Accordingly, the present invention provides a treatment of asthma, employing principles extracted from the plant *Murraya koenigii* in combination with other herbal extracts as described in claim 1. The invention also provides novel compositions useful in the treatment of asthma.

### Detailed description of the invention

A process for preparing an extract from the plant *Murraya koenigii,* useful in the treatment of asthma, said process comprising the steps of pulverising plant parts, extracting the plant pulverized parts with a solvent at ambient temperature, concentrating the extract by filtering and evaporating it under reduced pressure and lyophilizing the concentrate to obtain a lyophilized extract containing -active principles of the plant *Murraya koenigii.* is disclosed, but not part of the claimed invention.

In an embodiment, the plant materials are obtained from plant parts of *Murraya koenigii* selected from garden fresh leaves or leaves dried under shade.

In another embodiment, the leaves are pulverized by conventional methods to get homogenized leaves.

In yet another embodiment, the plant materials are extracted with solvents selected from hydrocarbon solvents, chlorinated solvents, ester solvents, ketonic solvents, alcohols, water and buffers. Thus, the solvents may be selected from the group consisting of petroleum ether (BP 40 - 60°C), petroleum ether (60°C - 80°C), benzene, pentane, hexane, chloroform, dichloromethane, carbon tetrachloride, diethyl ether, tetrahydrofuran, dioxane, acetone, cyclopentanone, ethyl acetate, ethyl formate, methanol, ethanol, n-butanol, water and buffers.

In another embodiment, the concentration of the extract is effected by filtering and evaporating the solvents under reduced pressure at a temperature range of 20°C - 80°C preferably at ambient temperature and lyophilizing the concentrate by conventional methods to obtain a mixture of the active principles.

In yet another embodiment, the extract obtained from the plant *Murraya koenigii* comprises active principles which appear as dark colored solids soluble in dimethylsulfoxide.

In another embodiment, the active principles obtained from the plant *Murraya koenigii* are biocompatible and non-toxic in nature. The active principles when subjected to chromatography are found to have R_{f} values 0.73, 0.60, 0.34 and 0.14 in chloroform and methanol in the ratio 19:1 and R_{f} values 0.60, 0.38, 0.24 and 0.15 in chloroform. The active principles exhibit four peaks with retention time of 3.37, 3.49, 4.0 and 5.69 methanol is used as solvent at 254nm. The extraction process is carried out for a period ranging from 1 - 120 hrs, preferably between 12-16 hrs.

In still another embodiment, plant material is extracted with appropriate solvents such as methanol or water or buffers in a percolator.

In yet another embodiment, the plant extract made by the methanol/appropriate solvent or water is concentrated under reduced pressure to obtain a mixture containing the active principles.

In another embodiment, the concentrate of the plant extract was lyophilized to render the principles substantially free of solvent or water.

In yet another embodiment, the lyophilized solid obtained as the mixture of active factors present in the leaf of *Murraya koenigii* is used for relief, treatment and cure of asthmatic problem.

### The method of preparation of the active factors comprises the following steps:

1) collecting the fresh leaves from the local suppliers,
2) drying the leaves under shade to a moderate degree or to take the fresh leaves as the starting material,
3) powdering the dried or homogenizing the fresh leaves,
4) adding the powder or homogenate in a percolator under the bulk of appropriate solvents; choosing hydrocarbon solvents such as petroleum ether (B.P 40-60°C), petroleum ether (B.P 60-80°C), pentane, hexane, benzene etc.; chlorinated solvents like chloroform, dichloromethane, carbon tetrachloride etc.; etheral solvents such as diethyl ether, tetrahydrofuran, dioxane etc.; ketonic solvents such as acetone, cyclopentanone etc.; ester solvents such as ethyl acetate, ethyl formate etc.; all alcohols such as methanol, ethanol, n-butanol etc.; water and buffers, for extraction,
5) extracting the percolated plant material using a percolator for a period ranging from 1 to 12 hours,
6) evaporating the solvent under reduced pressure at a temperature ranging from 20 to 80°C,
7) lyophilizing or drying the material,
8) storing the processed material in a cool and dry place in an air tight container, and
9) evaluating the bioactivity of the material.

Further, the invention provides pharmaceutical composition useful in the treatment of asthma, said composition comprising an effective amount of extract obtained from the plant *Murraya koenigii* together with, powder or extracts of plants selected from *M. paniculata Linn, H. abelmoschus, T. ammi, S. aromaticum, A. vasica Nees, E. hirta,* and *Murraya koenigii.* These additives are present in the range of 80-100 mg of *M. paniculata Linn,* 40-60 mg of *H. abelmoschus,* 38-62 mg of *T. ammi,* 7-13 mg of *S. aromaticum,* 85 - 115 mg of *A.vasica Nees* and 90-110 mg of *E.hirta.*

In an embodiment, the composition comprises :

| | |
|---|---|
| *M paniculata Linn. Syn. M exotica* | 90mg |
| (KAMINI) | |
| *H. abelmoschus* | 50mg |
| (JOWAN) | |
| *T. ammi* | 50mg |
| (LAVANGA) | |
| *S. aromaticum* | 10mg |
| (BASAK) | |
| *A. vasica Nees* | 100mg |
| (PUSITOA) | |
| *E.hirta* | 100mg |
| *Murraya koenigii* | |
| (Suravi Neem) | 100mg |

Further, the invention provides a treatment of asthma, said treatment comprising the steps of administering an effective amount of the composition to a subject in need thereof

In an embodiment, the lyophilized extract obtained from *Murraya Koenigii* is administered as described in claim 11.

In still another embodiment, the mode of administration is oral for the treatment of mild or acute asthma.

In yet another embodiment, the dosage level of the composition, comprising the extract from the plant *Murraya koenigii* is between 325-600 mg twice daily for the period ranging from 3 to 30 days.

In another embodiment, the dosage level is in between 325-600 mg twice daily for the period ranging from 3 to 15 days for mild asthmatic condition, and 15 - 30 days for acute asthmatic condition.

As said above, additives are present in a range of 80-100 mg of *M. paniculata Linn,* 40-60 mg of *H*. *abelmoschus,* 38-62 mg of *T. ammi,* 7-13 mg of *S. aromaticum, 85-*115 mg of *A.vasica Nees,* 90-110 mg of *E.hirta,* and 87-105 mg of *Murraya koenigii.* per dose.

The composition may be formulated in different physical forms suitable for oral or systemic use, although oral use is recommended.

In another embodiment, the additives obtained from the plants *M. paniculata Linn, H. abelmoschus, T. ammi, S. aromaticum, A. vasica Nees, E. hirta,* and *Murraya koenigii* are administered to impart properties such as antidiahorial, antiseptic, carminative, stimulation, anti-cough, anti- bronchitis and nourishment to the composition.

In another embodiment, the additives are obtained from : *M. paniculata Linn* (bark or root), *H. abelmoschus* from dried flower buds, *T. ammi* from leaves, *S. aromaticum* from whole plant *A.vasica Nees* from root, *E.hirta* from bark, and *Murraya koenigii* from leaves.

In addition, the invention provides an anti-oxidant composition for human beings and animals, said composition comprising a effective amount of extract obtained from the plant *Murraya koenigii* together with or optionally, associated with additives.

In still another embodiment, the additives comprise powder or extracts of plants selected from *M. paniculata Linn, H. abelmoschus, T. ammi, S. aromaticum, A.vasica Nees, E hirta,* and *Murraya koenigii.*

In an embodiment, the additives are present in the range of 80-100 mg of *M paniculate Linn,* 40-60 mg of *H. abelmoschus,* 38-62 mg of *T.ammi,* 7-13 mg of *S. aromaticum,* 85-115 mg of *A.vasica Nees,* 90-110 mg of *E hirta,* and 87-105 mg of *Murraya koenigii* per dose of the anti-oxidant composition.

As said above, the active factor(s) in *Murraya koenigii* are useful for relief, treatment and cure of asthmatic problem, the preparation of which comprises drying, powdering, and extracting the dried leaves of the plant, *Murraya koenigii,* in a percolator at an ambient temperature using appropriate solvents and concentrating the extract under reduced pressure and finally lyophilizing the concentrate to make the active factors.

The following examples are given by way of illustration and therefore should not be construed to limit the scope of the present invention.

### Collection of Plant material

The leaves of *M. koenigii* were collected from shrubs in the month of March-August 2000 from different areas of West Bengal, India. A voucher specimen is deposited at the department of Medicinal Chemistry at the Indian Institute of Chemical Biology, 4, Raja S. C. Mallick Road, Calcutta- 700 032, India.

### Extraction of the active principles from Murraya koenigii

### Example - 1

Fresh leaf containing branches of *Murraya koenigii* were collected, cleaned and washed with water and used as starting material. It was air-dried under shade and drying was continued till it was brittle to make it suitable for powdering in grinder-mixer.

87 gm. of the powdered leaves of *Murraya koenigii* was placed in a glass percolator (5-litr. capacity) with the addition of 1000 ml. of methanol so as to submerge the material satisfactorily. This was kept for 12 to 16 hrs (over night) at room temperature. Filtration of the extract through Whatman No. filter paper was carried out to collect the percolate. The process of extraction was repeated four times and the combined extract was evaporated to dryness under reduced pressure in a rotary evaporator keeping the temperature at 40°C (bath). The solid residual matter left was viscous in appearance and it was further dried by lypohilization. The yield was 14.84 gm. and the material was tested and found biologically active in both neutrophil or ex-vivo blood system.

### Example - 2

The air-dried leaves of *Murraya koenigii* weighing 90 gms., were powdered and taken in a percolator. It was dipped in 800-ml chloroform. The submerged material was allowed to be extracted by the solvent for 12 to 16 hours (over night). The extract was filtered through Whatman No.1 filter paper and collected; this process was repeated for three times. The extract was evaporated to dryness in flash evaporator under reduced pressure at 40°C. The residual substance was then dried in a desiccator under high vacuum and the solid mass weighing 11.5gm was tested and found biologically active.

### Example - 3

150 gm of fresh leaves of *Murraya koenigii* thoroughly washed in sterile water was homogenized with 750 ml of glass distilled water in a mixer-blender and then sonicated in an ultra sonic bath with 5 burst, each for 15min. Filtering through Whatman no. 1 filter paper was carried out to separate the material extracted in water. This type of treatment for extraction was repeated for three times. The combined extract was lyophilized yielding a powdered material, 1 lgm in weight. This was then tested for biological activity.

### Properties of the materials

The biologically active material obtained by examples 1 or 2 or 3 has the following properties:-
1. The dried solid prepared as stated above was a dark colored material soluble in DMSO or dimethyl sulfoxide.
2. Thin layer chromatography analysis of the active material shows four spots having R_{f}. values 0.60, 0.38, 0.24, and 0.15 respectively and R_{f} 0.73,0.60,0.34 and 0.14 in solvent system of chloroform and methanol taken in the ratio of 19:1.
3. The HPLC analysis of the active principles obtained from *Murraya koenigii* was carried out using Intersil ODS-3 (4.6x250mm) analytical column, with methanol as the solvent system and a flow rate 1.0ml /min. Detection at 254nm resolved the material into four peaks with the retention times 3.37, 3.49, 4.00, and 5.69 mins. respectively.

### EVALUATION OF ANTIOXIDANT ACTIVITY IN AN IN VITRO SYSTEM

Biological evaluation was carried out as per the following procedure.

### a) Inhibition of arachidonic acid oxidation in presence of the active material by neutrophil

Human peripheral blood neutrophil was prepared by standard method [Downey, G.P., Fukushima, T., Fialkow, L. and Waddel, T.K. (1995) Semin. Cell Biol. 6 345]. In brief, heparinised blood was treated with 2% gelatin (in normal saline) for half an hour to allow RBC separation at the bottom. The rest of the mixture was taken and subjected to gradient separation using Histopaque. The centrifugation was carried out at 1400 rpm at room temp. for 12 min. The neutrophil available as a separate layer at the bottom was collected and then made into suspension in sterile PBS or phosphate buffered saline.
(i) The neutrophil (1x104 /well of 24 well tissue culture plate) was treated with active material (20 g/ml) i.e. the extract obtained in Examples 1, 2 and 3 above, for 60 min., while the control did not contain any active material.
(ii) The neutrophil was primed with Phorbol Myristic Acetate (PMA) (10 M) for 30 min., followed by addition of active material (20 g/ml), or without it for 30 mins.
(iii) The neutrophil treated with active material (the extract) (20 g/ml) for 30 min. followed by PMA (10 M) activation for 30 min.

The oxygen consumption by the treated neutrophil was measured after ten minutes of the addition of arachidonic acid 10.2 µl (12.2 mg/ml in absolute alcohol) (Method. Enzymol. Ed. By Murphy, R.C, and Fitzpatrick F.A. Vol. 187 pp-268), in 2 ml volume of neutrophil suspension. The assay of the oxygen consumption was carried out by oxygen sensor (of Hansatec with O₂ electrode control having Clark type probe). The results of inhibition of oxygen consumption due to arachidonic acid oxidation are set out in Table-1

**Table-I**

| **Inhibition of arachidonic acid oxidation by neutrophil** | | | | |
|---|---|---|---|---|
| Treatment | | Micromole oxygen consumed/10 min/ | % of inhibition of O₂ consumption by active material with respect to | |
| | | | stimulation | without |
| | | | | stimulation |
| A .PBS 60 min. | | 16.08 | NA | --- |
| B Active extract 60 min in '1' | | 12.85 | ---- | 20 |
| | | 39.58 | ---- | |
| C. PMA 30 min. | | | | |
| | | 64.01 | ----- | |
| D. Calcium ionophore | | | | |
| | 30 min. | | | |
| E. Active extract 30min | | 21.42 | 46 | |
| | + PMA 30 min. | | | |
| | | 25.3 | 60 | |
| F. Active extract | | | | |
| | 30min.+Calcium ionosphere 30 min | | | |

Calcium ionophore gave better results as there was about 60% inhibition while PMA effects 46% inhibition of O₂ consumption by active material present in *Murraya koenigi*i leaf under in vitro neutrophil test. In both the stimulation remarkable inhibition of O₂ consumption indicates the efficacy of the material.

The results in Table-1 illustrated that the active factors in *Murraya koenigii* leaf acted as an inhibitor of oxygen consumption in presence of normal as well as activated human neutrophil and arachidonic acid. It suggests that oxidation of arachidonic acid is strongly inhibited by active factors present in *Miirraya koenigii* leaves. The leukotrienes are the biological mediators of asthma and the oxidation of arachidonic acid is the rate-determining step for the synthesis of the leukotrienes. The strategy of asthma drug development is to search or synthesize compounds or prepare substances that inhibit the synthesis of leukotrienes or precisely cause the inhibition of the oxidation of arachidonic acid. The active material or compounds prepared according to the process of the invention, not only inhibit the oxidation of arachidonic acid but are also free of toxicity and compatible for use in humans as the plant leaves are extensively used as food ingredients. Hence it qualifies as a drug for asthma.

### b) Inhibition of arachidonic acid oxidation in presence of the active material by Human ex vivo Blood.

500µl of heparinised human whole blood was taken in each well of a 6-well micrometer plate to which the *Murraya koenigi*i active material or none was added to have a final concentration of 50 gm/ml when the total volume was adjusted to 2 ml. with PBS. The plates were incubated for 2 hours with constant shaking. Next 10µl of arachidonic acid (12.2gm/ml in absolute alcohol stored under argon at -200C; Method. Enzymol. Ed. By Murphy, R C, and Fitzpatrick F.A. vol. 187 pp-268) was added to each well for 10 mins. Prior to the addition of Phorbol Myristic Acetate (PMA, 10(M) or calcium ionophore A23187 (20 gm/ml), (Spaethe S.M, Snyder D.W, Pechous P.A, Clarke T, VanAlstyne E.L Biochem. Pharmacy. 43; 1992,377-82). After 30 mins of the addition of the stimulator, a YSI Clark oxygen probe containing oxygen-monitoring equipment monitored oxygen consumption.

**Table-2**

| **Inhibition of arachidonic acid in whole human ex vivo blood in presence of*Murraya koenigii* leaf material** | | |
|---|---|---|
| Addition | Oxygen consumption (in nmol) /10 mins | Inhibition % of control . |
| A. None N 1 (PMA treated) | 39.99 | - |
| B. None N2 (Ca+ionophore) . | 62.41 I | |
| C. None N3 (patient's blood) | 32.15 | --- |
| D. Whole leaf in N1 blood | 22.1 1 | 45 |
| E. Water extract in N1 blood | 18.56 | 54 |
| F. Methanol extract in N1 blood | 10.70 | 73 |
| G. Methanol extract in N2 blood | 18.21 | 73 |
| H. Methanol extract in N3 blood | 11.70 | 70 |
| Chloroform extract in N1 blood | 11.11 | 72 |

| | | |
|---|---|---|
| * Patient having Eosinophil count 13 with 7 years of chronic asthma. | | |

The leaf extract of *Murraya koenigii* was also found to be highly effective in the nitro-blue Tetrazolium reduction test indicating that the extract is a powerful antioxidant.

Again, the Nitro Blue Tetrazolium (NBT) reduction test was carried out as per the standard procedure [Rouiller,Y.B., and Mauel J.(1987) Infec.Immun. S5, 587]. Neutrophil was prepared and treated with or without PMA (30 min.) and followed by leaf extract of *Murraya koenigii* (60 min.) or without it. Then 100(1 of NBT solution (3mg /ml) was added and cells were incubated for various intervals (0 to 120 mins). Formazan deposition was measured at 550 nm in an ELISA reader (Labsystem Multi Skan MS). The results showed that the activation of neutrophil with PMA was strongly inhibited about 6 times in the presence of the active material from the *Murraya koenigii* leaves at 120 min. The leaf preparation has strong antioxidant property which lend a firm support to recuperate from the condition that occurs during asthma.

### EVALUATION OF M. koenigii FOR ASTHMA

The leaves of these plants are frequently used as flavor enhancer in South Indian cooked food. However, heating of the leaves denatures its anti-asthmatic property. Its use as a cooking item or in food recipe has been known for centuries and no untoward occurrence is yet evident indicating that all components of this leaf are totally safe for human consumption. Use of this plant especially in South Indian food never produced bowel discomfort or vomiting or stomach upset or any other problem. Especially it is neither bitter in taste nor odd in taste. After taking the leaf preparation no bowel discomfort or vomiting or stomach upset or any gastrointestinal problem was reported by any of the healthy volunteers. From all these information it is quite convincing that any preparation from this leaf as a drug for asthma will be absolutely safe.

The extract obtained from the plant *Murraya koenigii* exhibits properties that make it useful in the treatment of asthma. Accordingly, compositions comprising the extract, preferably, the lyophilized extract can be used for the treatment of asthma in humans. The extract, when dried appears as dark solids. Pharmaceutical compositions comprising the extract of this plant along with additives, can be made in a variety of physical forms such as tablets, powders, syrups or other edible products. For the preparation of tablets, about 80-120mg of the extract from the plant is used. The tablets so prepared may be generally chewed or swallowed. The composition has no side effects and can be used without undue restriction. The composition was taken twice daily over a period of 30 days and found totally satisfactory without any complaints from any of the healthy volunteers.

An example of the composition prepared according to the invention is illustrated herein below. -

| LOCAL NAMES | PARTS SELECTED | AMOUNTS |
|---|---|---|
| *M. paniculata Linn. Syn. M exotica* | Bark of root | 80-100 |
| (KAMINI) | | |
| *H. abelmoschl*_{*l*}*s* | Seed | 40-60 |
| LATAKASTURI | | |
| *T.ammi* | | |
| JOWAN | Fruit | 3 8-62 |
| *S. aromaticum* | | |
| LAVANGA | Dried flower buds | 7-13 |
| *A. vasica Nees* | | |
| BASAK | Leaves | 85-115 |
| *E. hirta* | | |
| PUSITOA | Whole plant | 90-110 |
| *Murraya koerugii* | Root bark and leaves | 87-105 |

Below are representative examples of case studies wherein patients have been administered the composition of the invention. All these patients suffered from asthma, though the chronicity of the affliction varied from case to case.

Five volunteers (both female and male) were chosen with respect to their asthmatic condition depending on their sensitivity to the different causative agents that manifest their asthma.

### CASE STUDIES:

### Case-I

A female patient known for dust and cold sensitive severe asthma suffering for last seven years. Chest tightness, wheezing, cough, severe short-breath, night sleeplessness and high mucus secretion during late night hours were the primary symptoms. She had to use inhaler twice or thrice daily.

For medication two spoonful of whole plant preparation were taken by her twice daily for fifteen days. The observed effects of this medication: .
a) She did not have to use the inhaler except occasionally once in a day.
b) Wheezing and cough had totally stopped.
c) She was completely free from chest tightness.
d) She appeared physically normal and capable of strenuous activities like walking climbing up staircase to cross floors upto third level.

### Case - II

Two volunteer patients (one male and another female) with genetic predisposition for asthma suffering from sleeplessness during night time, very prone to asthmatic condition resulting from sensitively to pollution and shortness of breath they needed to take inhaler when there was a severe night attack They followed the medication as in case - 1 for one month.
i) Uptill the ninth month of observation they did not have any asthmatic attack,
ii) No shortness of breath was reported by them,
iii) In case of the male patient smoking was a risk enhancer for frequent episodes of asthmatic attack. But he did not have any breathing discomfort even if he smoked more than usual occasionally.
iv) Earlier the female patient had shortness of breath while taking long walks or climbing stair cases only upto a single floor but after the treatment she did not have any trouble as described above breathing even when she climbed upto third level.

### Case - III

Another young volunteer patient who suffers from periodical severe attack with acute shortness of breath and often had to be taken to hospital for fresh oxygen supply to ease out breathing acuteness.

During one such episode of acute attack of shortness of breath, he took the leaf preparation and the effects as observed were:
i) He did not have to go to the hospital,
ii) Since then he took this medication for a month, and
iii) His every day activities have increased remarkably and till date there has not been any occurrence of highly acute respiratory distress.

### Case-IV

An aged woman patient suffering from sleeplessness during night and extreme shortness of breath and showing no response to the modem medication including inhaler.

She was administered the leaf preparation which had to be taken twice daily for fifteen day and her condition has improved remarkably.

Thus, the application herein provides a simple fast and inexpensive process for preparation of a composition from an abundantly available plant for relief, treatment and cure of asthmatic problem satisfactorily. The method of administration of the plant material is also simple. The success rate is 100% among the volunteers. There has been no case of relapse during the observation time of 6 months. No adverse or side effects were observed after the treatment.

### The major advantages of the present invention are:

1. It is expected to provide relief, treatment and cure of asthmatic problem satisfactorily.
2. It is prepared from a plant material, which has been used in food for centuries and is biocompatible and non-toxic.
3. The plant is abundantly available.
4. Method of preparation of the active material /principle is simple, fast and inexpensive.
5. Its cultivation and propagation is easy as it grows an all kinds of soil.

## Claims

1. A pharmaceutical composition for the treatment of asthma, said composition comprising an effective amount of extract obtained from the plant *Murraya koenigii* together with powder or extracts of the plants *M.paniculata Linn, H. abelmoschus, T. ammi, S. aromaticum, A.vasica Nees* and *E.hirta.*

2. A composition as claimed in claim 1 wherein the powder or extracts are present in the range of 80-100 mg of *M.paniculata Linn,* 40-60 mg of *H. abelmoschus,* 38-62 mg of *T. ammi,* 7-13 mg of *S. aromaticum,* 85-115 mg of *A.vasica·Nees* and 90-110 mg of *E. hirta.*

3. A composition as claimed in claim 1 in tablet form wherein the extract of the plant *Murraya koenigii* is present in the range 80-120 mg.

4. A composition as claimed in claim 1 wherein the extract of the plant *Murraya koenigii* is present in the range of 87-105 mg per dose.

5. A composition as claimed in claim 1, wherein the powder or extracts are present in an amount 90 mg of *M.paniculata Linn,* 50 mg of *H. abelmoschus,* 50 mg of *T. ammi,* 10 mg of *S*. *aromaticum,* 100 mg of *A. vasica Nees* and 100 mg of *E.hirta* and 100 mg of *Murraya koenigii,* per dose.

6. A composition as claimed in claim 1 wherein the extract of the plant *Murraya koenigii* comprises active principles which are dark colored solids, soluble in dimethylsulfoxide.

7. A composition as claimed in claim 1 wherein the active principles obtained from the plant *Murraya koenigii* are biocompatible and non-toxic in nature.

8. A composition as claimed in claim 1 wherein the extract from the plant *Murraya koenigii* when subject to chromatography exhibits active principles having Rr values 0.73, 0.60, 0.34 and 0.14 respectively in chloroform.

9. A composition as claimed in claim 1 wherein the extract from the plant *Murraya koenigii* when subject to chromatography with methanol and chloroform in the ratio 19:1, exhibits R_{f} values 0.60, 0.38, 0.24 and 0.15 respectively.

10. A composition as claimed in claim 1 wherein the active principles in the extract from the plant *Murraya koenigii* exhibit four peak with retention times 3.37, 3.49, 4.0 and 5.69 respectively in methanol as solvent at 254nm.

11. Use of extract obtained from the plant *Murraya koenigii* together with powder or extracts of the plants *M.paniculata Linn, H. abelmoschus, T. ammi, S. aromaticum, A.vasica Nees* and *E.hirta,* for the manufacture of a pharmaceutical composition for the treatment of asthma.

12. Use as claimed in claim 11 wherein the composition is administered orally for treatment of mild or acute asthma.

13. Use as claimed in claim 11 wherein the dosage level of the composition is in between 325-600 mg twice daily for a period ranging from 3 to 30 days.

14. Use as claimed in claim 11 wherein the dosage level is in between 325-600 mg twice daily for a period ranging from 3 to 15 days for mild asthmatic condition, and 15-30 days for acute asthmatic condition.

15. Use as claimed in claim 11 wherein the powder or extracts are present in a range of 80-100 mg of *M.paniculata Linn,40-60* mg of *H. abelmoschus*,38-62 mg of *T. ammi,*7-13 mg of *S*. *aromaticum,* 85-115 mg of *A.vasica Nees,* 90-100 mg of *E.hirta,* and 87-105 mg of *Murraya koenigii,* per dose.

16. Use as claimed in claim 11 wherein the powder or extracts are present in an amount of 90 mg of *M.paniculata Linn,* 50 mg of *H. abelmoschus,* 50 mg of *T. ammi,* 10 mg of *S*. *aromaticum,*100 mg of *A. vasica Nees,*100 mg of *E.hirta* and 100 mg of *Murraya, koenigii* per dose.

17. Use as claimed in claim 11 wherein the the powder or extracts of *M.paniculata Linn, H. abelmoschus, T. ammi, S. aromaticum, A.vasica Nees, E.hirta* and *Murraya koenigii* are administered to include properties selected from antidiahorial; antiseptic, carminative, stimulation, anti-cough, anti-bronchitis and nourishment.

18. Use as claimed in claim 11 wherein the powder or extracts are obtained as: *M.paniculata Linn,* from bark or root, *H. abelmoschus* from dried flower buds, *T. ammi* from leaves, *S. aromaticum* from whole plant, *A.vasica Nees* from root, *E.hirta* from bark, and *Murraya koenigii* from leaves.

19. An anti-oxidant composition for human beings and animals, said composition comprising an effective amount of extract obtained from the plant *Murraya koenigii* together with powder or extracts of the plants *M.paniculata Linn, H. abelmoschus, T. ammi, S. aromaticum, A. vasica Nees* and *E.hirta.*

20. A composition as claimed in claim 19, wherein the powder or extracts are from *M. paniculata Linn, H. abelmoschus, T. ammi, S. aromaticum, A. vasica Nees, E. hirta,* and *Murraya koenigii* in the form of bark or root; seed; fruit; dried flower buds; leaves; whole plant; and root, bark, leaves, respectively.

## Patentansprüche

1. Pharmazeutische Zusammensetzung für die Behandlung von Asthma, wobei die Zusammensetzung eine wirksame Menge von Extrakt, erhalten aus der Pflanze *Murraya koenigii,* zusammen mit Pulver oder Extrakten der Pflanzen *M. paniculata Linn, H. abelmoschus, T. ammi, S. aromaticum, A. vasica Nees* und *E. hirta* umfaßt.

2. Zusammensetzung nach Anspruch 1, wobei das Pulver oder die Extrakte in dem Bereich von 80-100 mg von *M. paniculata Linn,* 40-60 mg von *H. abelmoschus,* 38-62 mg von *T. ammi,* 7-13 mg von *S. aromaticum,* 85-115 mg von *A. vasica Nees* und 90-110 mg von *E. hirta* vorhanden sind.

3. Zusammensetzung nach Anspruch 1 in Tablettenform, wobei der Extrakt der Pflanze *Murraya koenigii* in dem Bereich 80―120 mg vorhanden ist.

4. Zusammensetzung nach Anspruch 1, wobei der Extrakt der Pflanze *Murraya koenigii* in dem Bereich von 87-105 mg pro Dosis vorhanden ist.

5. Zusammensetzung nach Anspruch 1, wobei das Pulver oder die Extrakte in einer Menge von 90 mg von *M. paniculata Linn,* 50 mg von *H. abelmoschus,* 50 mg von *T. ammi,* 10 mg von *S. aromaticum,* 100 mg von *A. vasica Nees* und 100 mg von *E. hirta* und 100 mg von *Murraya koenigii* pro Dosis vorhanden sind.

6. Zusammensetzung nach Anspruch 1, wobei der Extrakt der Pflanze *Murraya koenigii* Wirkstoffe umfaßt, welche dunkel gefärbte Feststoffe, löslich in Dimethylsulfoxid, sind.

7. Zusammensetzung nach Anspruch 1, wobei die Wirkstoffe, erhalten aus der Pflanze *Murraya koenigii,* biokompatibel und nichttoxisch in der Beschaffenheit sind.

8. Zusammensetzung nach Anspruch 1, wobei der Extrakt aus der Pflanze *Murraya koenigii,* wenn der Chromatographie unterworfen, Wirkstoffe mit den R_{f}-Werten 0,73, 0,60, 0,34 bzw. 0,14 in Chloroform zeigt.

9. Zusammensetzung nach Anspruch 1, wobei der Extrakt aus der Pflanze *Murraya koenigii,* wenn der Chromatographie mit Methanol und Chloroform in dem Verhältnis 19:1 unterworfen, die R_{f}-Werte 0,60, 0,38, 0,24 bzw. 0,15 zeigt.

10. Zusammensetzung nach Anspruch 1, wobei die Wirkstoffe in dem Extrakt aus der Pflanze *Murraya koenigii* vier Peaks mit den Retentionszeiten 3,37, 3,49, 4,0 bzw. 5,69 in Methanol als Lösungsmittel bei 254 nm zeigen.

11. Verwendung von Extrakt, erhalten aus der Pflanze *Murraya koenigü,* zusammen mit Pulver oder Extrakten der Pflanzen *M. paniculata Linn, H. abelmoschus, T. ammi, S. aromaticum, A. vasica Nees* und *E. hirta* für die Herstellung einer pharmazeutischen Zusammensetzung für die Behandlung von Asthma.

12. Verwendung nach Anspruch 11, wobei die Zusammensetzung oral für die Behandlung von mildem oder akutem Asthma verabreicht wird.

13. Verwendung nach Anspruch 11, wobei das Dosierungsniveau der Zusammensetzung zwischen 325―600 mg zweimal täglich für einen Zeitraum, reichend von 3 bis 30 Tagen, liegt.

14. Verwendung nach Anspruch 11, wobei das Dosierungsniveau zwischen 325-600 mg zweimal täglich für einen Zeitraum, reichend von 3 bis 15 Tagen für mildes asthmatisches Leiden und 15-30 Tagen für akutes asthmatisches Leiden, liegt.

15. Verwendung nach Anspruch 11, wobei das Pulver oder die Extrakte in einem Bereich von 80-100 mg von *M. paniculata Linn,* 40-60 mg von *H. abelmoschus,* 38-62 mg von *T. ammi,* 7-13 mg von *S. aromaticum,* 85-115 mg von *A. vasica Nees,* 90-100 mg von *E. hirta* und 87-105 mg von *Murraya koenigii* pro Dosis vorhanden sind.

16. Verwendung nach Anspruch 11, wobei das Pulver oder die Extrakte in einer Menge von 90 mg von *M. paniculata Linn,* 50 mg von *H. abelmoschus,* 50 mg von *T. ammi,* 10 mg von *S. aromaticum,* 100 mg von *A. vasica Nees,* 100 mg von *E. hirta* und 100 mg von *Murraya koenigii* pro Dosis vorhanden sind.

17. Verwendung nach Anspruch 11, wobei das Pulver oder die Extrakte von *M*. *paniculata Linn, H. abelmoschus, T. ammi, S. aromaticum, A. vasica Nees, E. hirta* und *Murraya koenigii* verabreicht werden, um Eigenschaften, ausgewählt aus durchfallbekämpfend, antiseptisch, karminativ, zur Stimulation, gegen Husten, gegen Bronchitis und als Nahrungsmittel einzuschließen.

18. Verwendung nach Anspruch 11, wobei das Pulver oder die Extrakte als: *M. paniculata Linn,* aus Rinde oder Wurzel, *H. abelmoschus,* aus getrockneten Blütenknospen, *T. ammi,* aus Blättern, *S. aromaticum,* aus der gesamten Pflanze, *A. vasica Nees,* aus der Wurzel, *E. hirta* aus Rinde und *Murraya koenigii* aus Blättern erhalten werden.

19. Antioxidierend wirkende Zusammensetzung für Menschen und Tiere, wobei die Zusammensetzung eine wirksame Menge von Extrakt, erhalten aus der Pflanze *Murraya koenigii,* zusammen mit Pulver oder Extrakten der Pflanzen *M. paniculata Linn, H. abelmoschus, T. ammi, S. aromaticum, A. vasica Nees* und *E. hirta* umfaßt.

20. Zusammensetzung nach Anspruch 19, wobei das Pulver oder die Extrakte aus *M*. *paniculata Linn, H. abelmoschus, T. ammi, S. aromaticum, A. vasica Nees, E. hirta* und *Murraya koenigii* in der Form von Rinde oder Wurzel; Samen; Frucht; getrockneten Blütenknospen; Blättern; der ganzen Pflanze bzw. Wurzel, Rinde, Blättern sind.

## Revendications

1. Composition pharmaceutique pour le traitement de l'asthme, ladite composition comprenant une quantité efficace d'un extrait obtenu à partir de la plante *Murraya koenigii* accompagné d'une poudre ou d'extraits des plantes *M. paniculata Linn, H. abelmoschus, T. ammi, S. aromaticum, A. vasica Nees* et *E. hirta.*

2. Composition suivant la revendication 1, dans laquelle la poudre ou les extraits sont présents dans l'intervalle de 80-100 mg de *M. paniculata Linn,* 40-60 mg de *H. abelmoschus,* 38-62 mg de *T. ammi,* 7-13 mg de *S. aromaticum,* 85-115 mg de *A. vasica Nees* et 90-110 mg de *E. hirta.*

3. Composition suivant la revendication 1, sous forme de cachets, dans laquelle l'extrait de la plante *Murraya koenigii* est présent dans l'intervalle de 80-120 mg.

4. Composition suivant la revendication 1, dans laquelle l'extrait de la plante *Murraya koenigii* est présent dans l'intervalle de 87-105 mg par dose.

5. Composition suivant la revendication 1, dans laquelle la poudre ou les extraits sont présents dans une quantité de 90 mg de *M. paniculata Linn,* 50 mg de *H. abelmoschus,* 50 mg de *T. ammi,* 10 mg de *S. aromaticum,* 100 mg de *A. vasica Nees* et 100 mg de *E. hirta* et 100 mg de *Murraya koenigii,* par dose.

6. Composition suivant la revendication 1, dans laquelle l'extrait de la plante *Murraya koenigii* comprend des principes actifs qui sont des solides de couleur foncée, solubles dans le diméthylsulfoxyde.

7. Composition suivant la revendication 1, dans laquelle les principes actifs obtenus à partir de la plante *Murraya koenigii* sont biocompatibles et de nature non toxique.

8. Composition suivant la revendication 1, dans laquelle l'extrait issu de la plante *Murraya koenigii,* lorsqu'il est soumis à une chromatographie, affiche des principes actifs possédant des valeurs R_{f} de 0,73, 0,60, 0,34 et 0,14 respectivement dans le chloroforme.

9. Composition suivant la revendication 1, dans laquelle l'extrait issu de la plante *Murraya koenigii,* lorsqu'il est soumis à une chromatographie avec du méthanol et du chloroforme dans le rapport 19:1, affiche des valeurs R_{f} de 0,60, 0,38, 0,24 et 0,15 respectivement.

10. Composition suivant la revendication 1, dans laquelle les principes actifs dans l'extrait issu de la plante *Murraya koenigii* affichent quatre pics avec des temps de rétention de 3,37, 3,49, 4,0 et 5,69 respectivement dans le méthanol comme solvant à 254 nm.

11. Utilisation d'un extrait obtenu à partir de la plante *Murraya koenigii* accompagné d'une poudre ou d'extraits des plantes *M. paniculata Linn, H. abelmoschus, T. ammi, S. aromaticum, A. vasica Nees* et *E. hirta,* pour la fabrication d'une composition pharmaceutique pour le traitement de l'asthme.

12. Utilisation suivant la revendication 11, dans laquelle la composition est administrée oralement pour le traitement d'un asthme modéré ou aigu.

13. Utilisation suivant la revendication 11, dans laquelle le niveau de dosage de la composition est entre 325 et 600 mg deux fois par jour pendant une période variant de 3 à 30 jours.

14. Utilisation suivant la revendication 11, dans laquelle le niveau de dosage est entre 325 et 600 mg deux fois par jour pendant une période variant de 3 à 15 jours pour un état asthmatique modéré et de 15 à 30 jours pour un état asthmatique aigu.

15. Utilisation suivant la revendication 11, dans laquelle la poudre ou les extraits sont présents dans un intervalle de 80-100 mg de *M. paniculata Linn,* 40-60 mg de *H. abelmoschus,* 38-62 mg de *T. ammi,* 7-13 mg de *S. aromaticum,* 85-115 mg de *A. vasica Nees,* 90-100 mg de *E. hirta* et 87-105 mg de *Murraya koenigii,* par dose.

16. Utilisation suivant la revendication 11, dans laquelle la poudre ou les extraits sont présents dans une quantité de 90 mg de *M. paniculata Linn,* 50 mg de *H. abelmoschus,* 50 mg de *T. ammi,* 10 mg de *S. aromaticum,* 100 mg de *A. vasica Nees,* 100 mg de *E. hirta* et 100 mg de *Murraya koenigii,* par dose.

17. Utilisation suivant la revendication 11, dans laquelle la poudre ou les extraits de *M. paniculata Linn, H. abelmoschus, T. ammi, S. aromaticum, A. vasica Nees, E. hirta* et *Murraya koenigii* sont administrés pour inclure des propriétés choisies parmi anti-diarrhéique, antiseptique, carminative, stimulation, anti-toux, anti-bronchite et alimentation.

18. Utilisation suivant la revendication 11, dans laquelle la poudre ou les extraits sont obtenus sous la forme: *M. paniculata Linn* à partir d'écorce ou de racine, *H. abelmoschus* à partir de bourgeons de fleur séchés, *T. ammi* à partir de feuilles, *S. aromaticum* à partir de la plante entière, *A. vasica Nees* à partir de racine, *E. hirta* à partir d'écorce et *Murraya koenigii* à partir de feuilles.

19. Composition anti-oxydante pour les êtres humains et les animaux, ladite composition comprenant une quantité efficace d'un extrait obtenu à partir de la plante *Murraya koenigii* accompagné d'une poudre ou d'extraits des plantes *M. paniculata Linn, H. abelmoschus, T. ammi, S. aromaticum, A. vasica Nees* et *E. hirta.*

20. Composition suivant la revendication 19, dans laquelle la poudre ou les extraits sont issus de *M. paniculata Linn, H. abelmoschus, T. ammi, S. aromaticum, A. vasica Nees, E. hirta* et *Murraya koenigii* sous la forme d'écorce ou de racine; de graine; de fruit; de bourgeons de fleur séchés; de feuilles; de plante entière; et de racine, d'écorce, de feuilles, respectivement.
